(19) Europäisches Patentamt
European Patent Office
Office européen des brevets

(11) Publication number: **0 335 539 A2**

# (12) EUROPEAN PATENT APPLICATION

(21) Application number: 89302670.8

(22) Date of filing: 17.03.89

(51) Int. Cl.4: C07D 487/22 , A61K 31/40 , //(C07D487/22,257:00,209:00, 209:00,209:00,209:00)

(30) Priority: 28.03.88 JP 75292/88

(43) Date of publication of application: 04.10.89 Bulletin 89/40

(84) Designated Contracting States: CH DE FR GB IT LI

(71) Applicant: HAMARI YAKUHIN KOGYO KABUSHIKI KAISHA also known as HAMARI CHEMICALS LTD.
4-29, Kunijima 1-chome
Higashi-yodogawa-ku
Osaka 533(JP)

(72) Inventor: Uchimoto, Mari
417-1, Konoike Higashiosaka
Osaka 578(JP)
Inventor: Suganuma, Michito
9-1, Takadamachi
Yamatokoriyama Nara 639-11(JP)
Inventor: Kawabe, Hirofumi
8-7, Sakuradai 4-chome
Nerima-ku Tokyo 176(JP)
Inventor: Otani, Takuzo
3-17-204, Shinhinoodai 3-cho Sakai
Osaka 590-01(JP)
Inventor: Aizawa, Katsuo
26-20, Tomiokanishi 6-chome Kanazawa-ku
Yokohama Kanagawa 236(JP)

(74) Representative: Burford, Anthony Frederick et al
W.H. Beck, Greener & Co. 7 Stone Buildings
Lincoln's Inn
London WC2A 3SZ(GB)

## (54) Porphyrin derivatives.

(57) Porphyrin derivatives of the formula A:

EP 0 335 539 A2

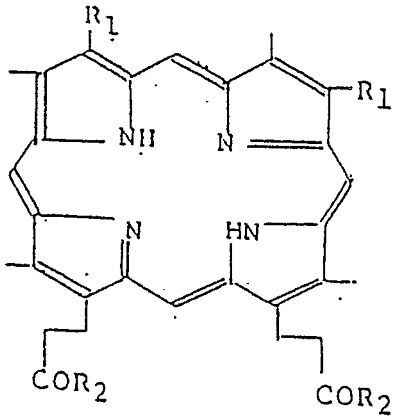

(A)

wherein each $R_1$ independently is a hydrogen atom, a $C_1$-$C_4$ alkyl group, an ethenyl group, or a 2-hydroxyethyl group; one $R_2$ is a hydroxyl group and the other $R_2$ is

$$NH(C_mH_{2m})N\genfrac{}{}{0pt}{}{A}{B} \quad \text{or} \quad NH(C_mH_{2m})\overset{+}{N}\text{-}\genfrac{}{}{0pt}{}{A}{\genfrac{}{}{0pt}{}{B}{C}}.X^-,$$

(each A, B, and C is independently a $C_1$-$C_4$ alkyl group; $X^-$ is an halogen or organic acid ion; and m is an integer of 1 to 23, and pharmaceutically acceptable salts are photosensitive, less accumulable in the skin and specifically accumulative in cancer tissues as compared with known porphyrins. They can be obtained from the corresponding diester compounds.

## PORPHYRIN DERIVATIVES

The present invention relates to novel porphyrin derivatives employed in the diagnosis and treatment of cancer.

Photodynamic diagnosis and therapy, which comprises administering intravenously a photosensitive substance showing the ability of accumulating in cancer cells and, after the substance is accumulated in the cells, diagnosing and treating cancer tissues by the irradiation of laser light, is achieving brilliant success in the diagnosis and treatment of early cancer. (T.J. Dougherty, "Photodynamic Therapy of Tumors and other Diseases" pp. 267-279, (1985)). As the photosensitive substance for this purpose, there have been mainly used hematoporphyrin (hereinafter abbreviated as HP) or hematoporphyrin derivatives (hereinafter abbreviated as HpD) produced by treating HP with sulfuric acid in acetic acid and then subjecting the resultant to alkali hydrolysis, followed by neutralization.

However, it is difficult to obtain HP as a pure substance (D. Dolphin ed. "The Porphyrins" Vol. 1, pp. 297-298 (1978)). In addition, HpD is a mixture of dozens of kinds of porphyrin, because it is produced with the use of such HP, and postulated structures have only been proposed for its active form. It is also known that the above-mentioned substances are accumulated not only in cancer tissues but also in normal tissues, particularly the liver and the skin in large quantities.

These constitute great obstacles in the clinical application of the substances.

The present inventors previously obtained porphyrin derivatives having two aminium groups in the side chains as photosensitizing substances which have affinity to cancer cells (Japanese Patent Application Laid-Open No. 167783/1987). Successively they researched photosensitizing substances which accumulate specifically in cancer tissues, and found that porphyrin derivatives having one aminium group in the side chains are less accumulable in the skin, and come to establish the present invention.

According to the present invention there is provided porphyrin derivatives of the general formula A:

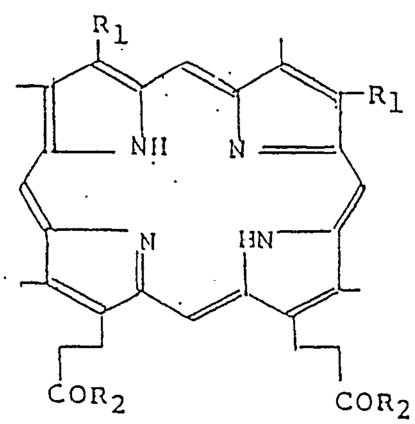

(A)

wherein each $R_1$ independently is a hydrogen atom, a $C_1$-$C_4$ alkyl group, an ethenyl group, or a 2-hydroxyethyl group, one $R_2$ is a hydroxyl group and the other $R_2$ is

$$NH(C_mH_{2m})N\genfrac{}{}{0pt}{}{A}{B} \quad \text{or} \quad NH(C_mH_{2m})\overset{+}{N}\genfrac{}{}{0pt}{}{A}{\genfrac{}{}{0pt}{}{B}{C}}\cdot X^-;$$

(each A, B, and C independently is a $C_1$-$C_4$ alkyl group; $X^-$ is a halogen or organic acid ion; and m is an integer of 1 to 23, and pharmaceutically acceptable salts thereof.

Preferable examples of alkyl groups represented by $R_1$, A, B and C are methyl, ethyl, and n-propyl groups. As a halogen ion represented by $X^-$, chlorine, bromine or iodine is preferable. Examples of organic ions are preferably the ions derived from acetic acid or p-toluenesulfonic acid.

The porphyrin derivatives of the present invention each have a carboxyl group and, as the case may be,

$$-CO(C_mH_{2m})N\begin{smallmatrix} A \\ B \end{smallmatrix}.$$

Therefore, they may form salts with a base or an acid and may be used in free form or as a pharmaceutically acceptable salt, for example, a salt with a base such as a sodium or potassium salt, or a salt with an acid such as a hydrochloride acid salt.

The porphyrin derivatives can be synthesized, for example, according to a pathway briefly described below:

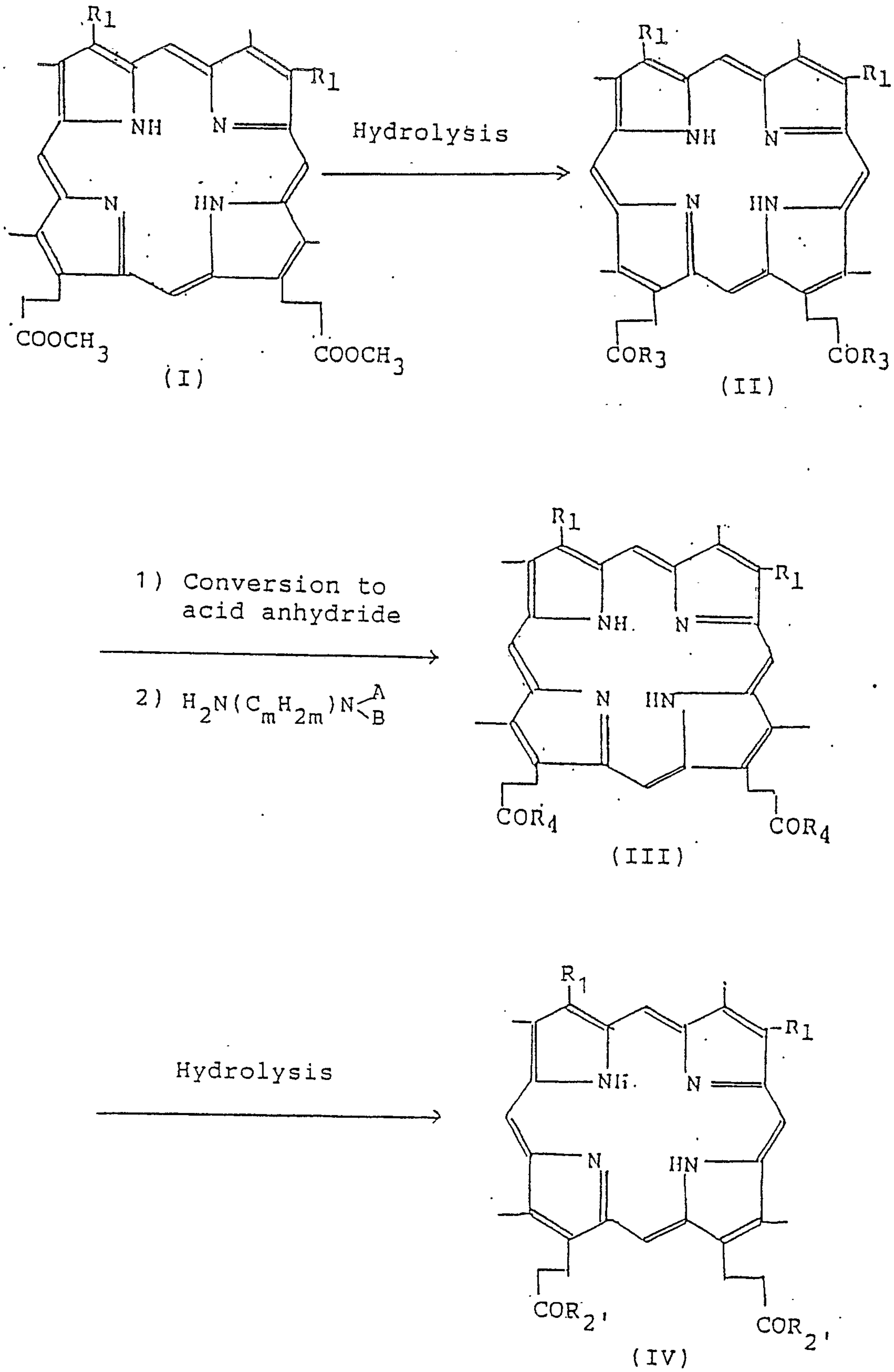
Hydrolysis
1) Conversion to acid anhydride
2) $H_2N(C_mH_{2m})N<^A_B$
Hydrolysis

(I)

(II)

(III)

(IV)

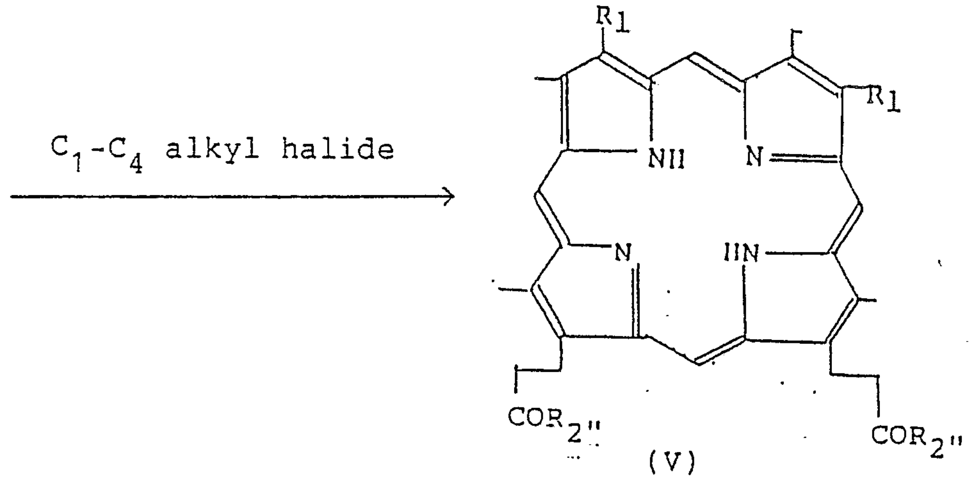


(V)

In Formulae I to V above, $R_1$ is as defined above in connection with Formula A, one of $R_3$ is an hydroxyl group and the other of $R_3$ is a methoxy group; one of $R_4$ is a methoxy group and the other of $R_4$ is

$$NH(C_mH_{2m})N\langle^{A}_{B},$$

wherein A, B and m are as defined above in connection with Formula A; one of $R_2'$ is an hydroxyl group and the other $R_2'$ is

$$NH(C_mH_{2m})N\langle^{A}_{B};$$

and one of $R_2''$ is an hydroxyl group and the other of $R_2''$ is

$$NH(C_mH_{2m})\overset{+}{N}\langle^{A}_{C}{-}B \cdot X^-,$$

wherein A, B, C and m are as defined above in connection with Formula A.

Namely, the porphyrin derivatives represented by the general formula (II) can be obtained by hydrolyzing the porphyrin derivatives represented by the general formula (I) with an acid or alkali by a conventional procedure. Usually, as the acid utilized in the hydrolysis, there may be exemplified a mineral acid such as hydrochloric acid or sulfuric acid or an organic acid such as acetic acid or p-toluenesulfonic acid, and as the alkali, there may be exemplified sodium hydroxide, potassium hydroxide and ammonia. Sodium hydroxide is preferably employed. The reaction is completed at 0° to 150°C in 5 minutes to 48 hours. For example, in the case of employing sodium hydroxide, the reaction is completed at 100° to 120°C in 30 minutes.

The porphyrin derivatives represented by the general formula (II) are reacted in a solvent with an agent which converts a carboxyl group to its anhydride in the presence of an acid-capturing agent to convert the derivatives to the corresponding acid anhydride compounds, and then reacted with a compound represented by the general formula

$$H_2N(C_mH_{2m})N\langle^{A}_{B},$$

(wherein each symbol has the same definition as above) to obtain the compounds represented by the general formula (III). In this reaction, solvents such as anhydrous tetrahydrofuran, dioxane, ethyl acetate, or

methylene chloride may be employed, preferably methylene chloride. As the acid-capturing agent, triethylamine or tributylamine is usually employed. As the agent which converts a carboxylic acid to its anhydride, an acid chloride or alkyl chloroformate usually is employed, preferably pivaloyl chloride.

Suitable temperatures and times are selected for the reaction. It is better to avoid extremely high temperature, because it may cause unfavourable side reaction. In the case of employing methylene chloride as the solvent, triethylamine as the acid-capturing agent and pivaloyl chloride as the agent which converts a carboxylic acid to its anhydride, the reaction is completed at below 0° C in several minutes to 2 hours, and the amidation can be completed at room temperature in several hours to overnight.

The porphyrin derivatives represented by the general formula (IV) and their salts are obtained by hydrolyzing the porphyrin derivatives represented by the general formula (III) making use of equimolar alkali by a conventional procedure. As the alkali employed in the hydrolysis, there usually is exemplified sodium hydroxide, potassium hydroxide, or ammonia. Sodium hydroxide is preferably employed. This reaction is completed at 0° to 150° C in 5 minutes to 48 hours. For example, in the case of employing sodium hydroxide, the reaction finishes at 40° to 60° C in 1 to 2 hours.

The porphyrin derivatives represented by the general formula (V) and their carboxylic acid salts are obtained by reacting the porphyrin derivatives represented by the general formula (IV) or their salts with a $C_1$-$C_4$ alkyl halide in the presence or absence of a solvent. As preferable solvents employed in this reaction, there may be exemplified methylene chloride, chloroform, ethylene dichloride, and benzene. Suitable temperatures and times are selected for the reaction. The reaction is completed usually at 0° to 100° C in several minutes to overnight. For example, in the case of employing benzene as a solvent, the reaction is complete at 20 to 30° C in 30 minutes to 1 hour.

The post-treatment and purification of these novel porphyrin derivatives may be carried out by usual procedures, for example, extraction, recrystallization, or column chromatography.

The compounds according to present invetnion have the following merits in the diagnosis and therapy of cancer.

After administering intravenously to cancer-carrying animals, they accumulate specifically in cancer tissues and emit a characteristic fluorescent spectrum when irradiated by light of an appropriate wavelength. This enables the mere measurement of such flourescent spectra to determine the size and location of cancer.

When irradiated by laser light of a suitable wavelength, they generate singlet oxygen which has cell-killing action, causing selective necrosis in cancer tissues alone without inflicting damage to normal tissues.

Various methods of administrating the present compounds can be employed. For example, they may be administered intravenously, subcutaneously, intraperitoneally, orally and intrarectally. They are normally administered in an amount of 0.1 to 350 mg per dose, however, the doses are not limited to such range, but variable according to the manner of therapy.

The following are an experiment example and examples to illustrate present invention in detail, however, the present invention is not limited by these examples.

## Experimental Example

The affinity of the present porphyrin derivatives to cancer tissues was tested by the following method while using mice transplanted with cancer cells.

### Experimental method:

Three week old mice of the Balb/c strain were transplanted subcutaneously with mouse kidney fibrosarcoma (MKSA) cells (1 x $10^7$ cells). 2 to 3 weeks after the transplantation, each of the test samples was administered to mice into the tail vain at a dose of 20 mg per kg body weight. 24 hours after the administration, cancer cells and organs were excised, and the amount of characteristic fluorescence emitted from the compound accumulated in each tissue was measured by an endoscopic diagnosis system using an excimer dyelaser (K. Aizawa et al., Journal of Laser Medical Association of Japan (Rehzah Igakukaishi), vol. 5, pp. 63-68 (1984)).

### Test Samples:

I. Sodium 7,12-diethenyl-3,8,13,17-tetramethyl-2-(N-(2-trimethylammonioethyl)carbamoyl)ethyl-21H, 23H-porphine-18 propionate.iodide and sodium 7,12-diethenyl-3,8,13, 17-tetramethyl-18-H-(N-2-trimethylammonioethyl) carbamoyl)ethyl-21H,23H-porphine-2-propionate.iodide

II. 7,12-diethenyl-3,8,13,16-tetramethyl-2,18-bis(2-(2-trimethylammonioethyloxy)carbonylethyl)-21H,23H-porphine.diiodide

III. 7,12-diethenyl-3,8,13,17-tetramethyl-2,18-bis(2(N-(2-trimethylammonioethyl)carbamoyl)ethyl)-21H,23H-porphine.diodide

IV. Hematoporphyrin IX

V. HpD

Results:

Shown in Table 1 are the amounts of fluorescence in cancer tissues and ratios of the amounts of fluorescence in cancer tissues to that in normal tissues.

Table 1

| Compound | Strength of fluorescence in tumour | Ratio of strength of fluorescence (organ/tumour) | | | |
|---|---|---|---|---|---|
| | | Skin | Lung | Liver | Kidney |
| I | 14.55 | 0.34 | 0.07 | 0.07 | 0.00 |
| II | 15.08 | 0.47 | 0.01 | 0.09 | 0.00 |
| III | 14.89 | 0.74 | 0.07 | 0.04 | 0.00 |
| IV | 5.7 | 0.84 | 0.01 | 0.09 | 0.00 |
| V | 3.45 | ---- | 0.45 | 0.45 | 0.45 |

Example 1

Synthesis of sodium 7,12-diethenyl-3,8,13,17-tetramethyl-2-[N-(2-dimethylaminoethyl)carbamoyl]ethyl-21H,23H-porphine-18-propionate and sodium 7,12-diethenyl-3,6,13,17-tetramethyl-18-[N-(2-dimethylaminoethyl)carbamoyl]ethyl-21H,23H-porphine-2-propionate:

Into 400 ml of pyridine, 10 g of methyl 7,12-diethenyl-3,8,13,17-tetramethyl-21H,23H-porphine-2,18-dipropionate was added. After adding 200 ml of water and 20 ml of aqueous 1 N sodium hydoxide thereto, the solution was refluxed for 1 hour, and the reaction mixture was concentrated at 40 °C under reduced pressure. The residue was washed with 500 ml of chloroform and then dissolved in 500 ml of water. To the solution, 20 ml of acetic acid was added, and resultant precipitates were collected by filtration and dried at 40 °C for 8 hours to obtain 8.7 g of dark blackish brown crude product.

In 200 ml of methylene chloride, 6.7 g of the crude product was suspended, 6 ml of triethylamine was added to the suspension, and the mixture was cooled on a ice bath to -10 °C. To the cooled mixture, 4 ml of pivaloyl chloride was added and the mixture was stirred at -10 °C to 0 °C for 4 hours. After adding 6 ml of N,N-dimethylethylenediamine, the mixture was further stirred at room temperature overnight. To the reaction mixture, 400 ml of methylene chloride and saturated aqueous sodium chloride solution were added. The organic layer was separated, washed with each 200 ml of saturated aqueous sodium chloride solution three times, and dried over anhydrous magnesium sulfate, and then solvent was removed by distillation from the organic layer to give 7.2 g of crude product as dark blackish brown oily substance. This oil was purified by column chromatography on 500 g of alumina (activity V) using methylene chloride as an eluent to give 1.1 g of methyl 7,12-diethenyl-3,8,13,17-tetramethyl-2-[N-(2-dimethylaminoethyl)carbamoyl]ethyl-21H,23H-porphine-18-propionate and methyl 7,12-diethenyl-3,8,13,17-tetramethyl-18-[N-(2-dimethylaminoethyl)carbamoyl]ethyl-21H,23H-porphine-2-propionate as blackish brown crystals.

IR(cm$^{-1}$): 2580, 2800, 2750, 1730, 1650.

NMR ($CDCl_3$): =9.25, 9.16, 9.12, 8.98 (s, 1Hx4), 7.45-7.86(m, 2H), 5.70-6.10 (m, 6H), 3.90 (broad, 4H,) 3.38 (s, 3H), 2.60-3.20 (m, 17H), 1.62 (s, 6H), -5.08 (broad, 2H)

By adding 150 ml of pyridine, the crystals were dissolved and aqueous 1 N sodium hydroxide solution was added thereto. The mixed solution was stirred on a hot water bath at 80 °C for 1 hour, and then solvent was evaporated under reduced pressure to give 1.2 g of the product as blackish brown crystals.
IR ($cm^{-1}$): 2850, 2800, 1630, 1470.
UV/VIS (in Py): 405.4, 505.2, 541.2, 574.8, 628.2 nm.

Example 2

Synthesis of sodium 7,12-diethenyl-3,8,13,17-tetramethyl-2-[N-(2-trimethylammonioethyl)carbamoyl]ethyl-21H,23H-porphine-18-propionate iodide and sodium 7,12-diethenyl-3,8,13,17-tetramethyl-18-[N-(2-trimethylammonioethyl)carbamoyl]ethyl-21H,23H-porphine-2-propioninate iodide

Into 200 ml of methanol, 1.2 g of the mixture of sodium 7,12-diethenyl-3,8,13,17-tetramethyl-2-[N-(2-dimethylaminoethyl)carbamoyl]ethyl-21H,23H-porphine-18-propionate and sodium 7,12-diethenyl-3,8,13,17-tetramethyl-18-[N-(2-dimethylaminoethyl)carbamoyl]ethyl-21H,23H-porphine-2-propionate was dissolved, 2 ml of methyl iodide was added thereto, and allowed to stand at room temperature overnight, and then the solvent was evaporated under reduced pressure. The residue was dried under reduced pressure at room temperature for 8 hours to give 1.5 g of the product as dark blackish brown crystals.
IR ($cm^{-1}$): 2850, 2825, 1650
UV/VIS (in methanol): 396.6, 502.8, 537.2, 573.0, 627.0 nm.

**Claims**

1. A porphyrin derivative of the formula;

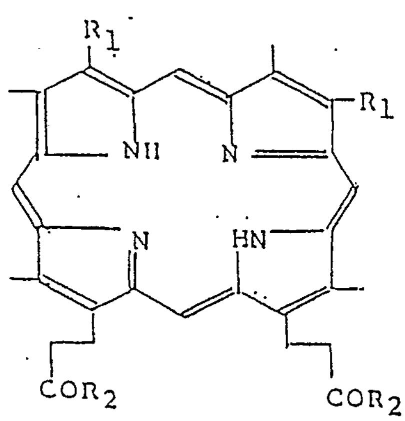

wherein $R_1$ is hydrogen atom, a $C_1$-$C_4$ alkyl group, ethenyl group, or 2-hydroxyethyl group; in a pair of $R_2$, one is hydroxyl group and the other is

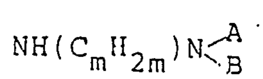 or

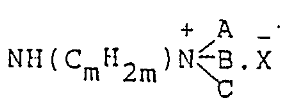

(wherein A, B or C is independently a $C_1$-$C_4$ alkyl group; $X^-$ is an ion of halogen or an organic acid; and m is an integer of 1 to 23,) or a pharmaceutically acceptable salt thereof.

2. A porphyrin derivative as claimed in claim 1, which is 7,12-diethenyl-3,8,13,17-tetramethyl-2-[N-(2-dimethylaminoethyl)carbamoyl]ethyl-21H,23H-porphine-18-propionic acid,

7,12-diethenyl-3,8,13,17-tetramethyl-18-[N-(2-dimethylaminoethyl)carbamoyl]ethyl-21H,23H-porphine-2-propionic-acid,
7,12-diethenyl-3,8,13,17-tetramethyl-2-[N-(2-trimethylammonioethyl)carbamoyl]ethyl-21H,23H-porphine-18-propionic acid.halide,
7,12-diethenyl-3,8,13,17-tetramethyl-18-[N-(2-trimethylammonioethyl)carbamoyl]ethyl-21H,23H-porphine-2-propionic acid.halide, or a salt thereof.

3. A composition for diagnosing and/or treating cancer which comprises a porphyrin derivative as claimed in Claim 1 or Claim 2 and a pharmaceutically acceptable diluent or carrier therefor.

4. A porphyrin derivative or salt thereof as claimed in Claim 1 or Claim 2, for use in the diagnosis and/or treatment of cancer.

5. The use of porphyrin derivative or salt thereof as claimed in Claim 1 or Claim 2 in the manufacture of an composition for use in the diagnosis and/or treatment of cancer.